# EUROPÄISCHE PATENTSCHRIFT

(11) **EP 0 679 247 B1**
(45) Veröffentlichungstag und Bekanntmachung des Hinweises auf die Patenterteilung: **15.04.1998**
(21) Anmeldenummer: 94931587.3
(22) Anmeldetag: 08.11.1994
(51) Int. Cl.: G01G 23/00, G01G 17/04, A61B 5/20

(54) **URINMESSGERÄT UND VERFAHREN ZUM ERMITTELN DER DICHTE VON URIN**
URINE MEASURING DEVICE AND PROCESS FOR DETERMINING URINE DENSITY
APPAREIL DE MESURE ET PROCEDE PERMETTANT DE DETERMINER LA DENSITE DE L'URINE

(30) Priorität: 12.11.1993 DE 4338687
(43) Veröffentlichungstag der Anmeldung: 02.11.1995
(73) Patentinhaber: Fresenius AG, 61350 Bad Homburg v.d.H (DE)
(72) Erfinder: WESTPHAL, Detlef, D-61440 Oberursel (DE); Metzner, Klaus, D-61381 Friedrichsdorf (DE); SCHMEHL, Wolfgang, D-61348 Bad Homburg (DE); GRIMM, Günther, D-61352 Bad Homburg (DE); LAPP, Uwe, D-35644 Hohenahr-Mudersbach (DE); WILD, Andreas, D-61169 Friedberg (DE); KOERDT, Franz-Wilhelm, D-60437 Frankfurt am Main (DE)
(74) Vertreter: Luderschmidt, Schüler & Partner GbR
(86) Internationale Anmeldenummer: EP9403671
(87) Internationale Veröffentlichungsnummer: WO9513524

(56) Entgegenhaltungen:
- EP-A- 0 235 386
- WO-A-92/18856
- DE-A- 3 544 031
- FR-A- 2 639 931
- US-A- 3 859 854
- US-A- 4 712 567

## Beschreibung

Die Erfindung betrifft ein Urinmeßgerät zum automatischen Überwachen und Erfassen des Urinflusses eines katheterisierten Patienten gemäß der DE-A-35 44 031.

Auf Intensivstationen in Krankenhäusern ist es notwendig, den Flüssigkeitshaushalt eines Patienten zu bilanzieren, indem dem Patienten beispielsweise mittels Infusionen zugeführte Flüssigkeitsmengen erfaßt und mit den Flüssigkeitsmengen verglichen werden, die den Körper des Patienten auf natürlichem Wege oder über künstliche Kreisläufe verlassen. Die wichtigste Körperflüssigkeit, die den Körper des Patienten regelmäßig verläßt, ist der Harn oder Urin, der auf Intensivstationen üblicherweise aus der Blase des Patienten abgeführt wird, indem ein Katheter, d.h. ein röhrenförmiges Ableitelement, in die Blase des Patienten geschoben wird. An den Katheter schließt sich üblicherweise ein Abflußschlauch an, durch den der Urin in ein unterhalb des Patienten am Bett befestigtes Sammelgefäß abläuft. Um eine retrograde Verkeimung der Schlauchleitung und damit eine Infektion des Patienten zu verhindern, ist innerhalb der Schlauchleitung häufig eine Tropfkammer angeordnet, durch die der Verkeimungsweg unterbrochen wird. Zu den wichtigsten aussagekräftigen Meßwerten, an denen ein diagnostizierender Arzt im Zusammenhang mit Harn interessiert ist, gehört das Gesamtvolumen des innerhalb eines bestimmten Zeitraumes ausgeschiedenen Harnes, wie beispielsweise der sogenannte 24-Stunden-Harn, der durchschnittliche Volumenfluß an Harn innerhalb eines bestimmten Zeitintervalls, die spezifische Dichte des Harns sowie eine optische Kontrolle der Farbe des Urins.

Zu den traditionell verwendeten Vorrichtungen gehören solche, wie sie beispielsweise in der EP-A-0 008 450 beschrieben sind. Der dort beschriebene durchsichtige Urinsammelbeutel weist eine Skalierung auf, anhand derer das gesammelte Gesamtvolumen des Urins ermittelbar ist. Weiterhin ist es in vielen Krankenhäusern nach wie vor üblich, daß der durchschnittliche Urinfluß stündlich ermittelt wird, indem eine Krankenschwester oder ein Pfleger alle Stunde den Volumenzuwachs an Urin feststellt und in eine Liste einträgt.

Weiterhin erlaubt der durchsichtige Beutel gemäß dem Stand der Technik eine optische Kontrolle der Farbe des Urins, was allerdings dadurch erschwert wird, daß der vollständig aus durchsichtigem Material gefertigte Sammelbeutel zum Teil unterhalb des Bettes und damit vor einem Hintergrund einheitlicher Farbe aufgehängt ist, und zudem so tief hängt, daß sich der kontrollierende Arzt oder Pfleger bücken muß.

Die Ermittlung der Dichte des Urins erfolgte in der Vergangenheit häufig über Teststäbchen, deren Ende mit einer Substanz beschichtet ist, die auf die in dem Urin enthaltenen Elektrolyte anspricht und einen Farbindikator zum Umschlag bringt. Wenngleich die so ermittelte Dichte des Urins relativ ungenau ist, so ergeben sich für den behandelnden Arzt doch aufschlußreiche Anhaltspunkte für die Funktion der Niere des Patienten. Die Dichte läßt sich so ermitteln, da die Dichte des Urins wegen der schweren Cl⁻-Ionen in erster Linie durch die Menge an im Urin enthaltenen Elektrolyten beeinflußt wird und weniger durch die ebenfalls im Urin enthaltenen Nicht-Elektrolyten, zu denen insbesondere Harnstoff gehört.

Es ist offensichtlich, daß die oben beschriebene manuelle Methode sehr aufwendig ist und darüber hinaus den Nachteil mit sich bringt, daß eine Beobachtungsperson zu bestimmten Zeitpunkten den Urinsammelbeutel kontrollieren muß. Bei dem auf Intensivstationen im allgemeinen ständig herrschenden Zeitdruck und Personalmangel kommt es hierbei mitunter zu vergessenen Messungen etc., so daß die beschriebene Methode insgesamt unzufriedenstellend ist.

Aus der EP-A-0 471 413 ist ein weiteres Beispiel für einen durchsichtigen Urinsammelbeutel bekannt, mit dem lediglich eine manuelle Erfassung der gewünschten Kennwerte möglich ist.

Es hat daher in der Vergangenheit nicht an Vorschlägen gefehlt, wie sich insbesondere das gesammelte Gesamtvolumen des Urins automatisch erfassen läßt und Werte für den durchschnittlichen Volumenfluß an Urin innerhalb eines bestimmten Zeitintervalls bestimmt werden können.

Den meisten Lösungsvorschlägen liegt dabei die Idee zugrunde, das in dem Urinsammelbeutel gesammelte Urinvolumen mittels einer physikalischen Hilfsgröße zu erfassen.

Ein Beispiel für eine solche Vorrichtung findet sich in der DE-PS 32 40 191, in der die Lage des Flüssigkeitsspiegels in dem Urinsammelgefäß mittels einer Ultraschallquelle erfäßt werden soll. Diese Technik ist aufwendig und fehleranfällig, da einerseits ein kleiner relativer Fehler bei der Bestimmung des Flüssigkeitsspiegelstandes einen verhältnismäßig großen absoluten Fehler bei der Volumenbestimmung nach sich zieht, und weiterhin sichergestellt sein muß, daß der Flüssigkeitsspiegel senkrecht zu einer definierten Achse verläuft, d.h. es muß sichergestellt sein, daß das Gerät nicht gekippt ist. Gerade die letztere Forderung läßt sich auf Intensivstationen häufig nicht verwirklichen.

Aus der DE-A-40 23 336 ist ein Gerät zur Überwachung von Urin bekannt, bei dem das Füllvolumen des in einer Meßkammer gesammelten Urins kapazitiv gemessen wird, wobei der Flüssigkeitsstand an Urin die Kapazität eines Meßkondensators beeinflußt. An dieser Technik ist nachteilig, daß sich eine solche Bauweise nur schwer für ein Wegwerfteil eignet, da dies zu teuer ausfallen würde. Bei dauerhafter Verwendung eines solchen Systems ist es notwendig, die Meßkammer und den Meßkondensator häufig zu reinigen, da es ansonsten zu Verfälschungen des Meßergebnisses aufgrund von sich bildenden Ablagerungen kommen kann. Generell läßt sich sagen, daß sich die meisten vorgeschlagenen Systeme zur Überwachung eines Urinflusses nicht zur Konstruktion eines billigen Einwegteiles eignen, das nach Gebrauch entsorgt werden kann, da die verwendeten physikalischen Prinzipien Meßsensoren voraussetzen, die mit dem Urin selbst direkt in Kontakt kommen.

Aus der US-A4,343,316 und der EP-A-0 109 373 sind zwei Beispiele für ein weiteres im Stand der Technik verwendetes Meßprinzip bekannt, bei der im ersten Fall ein durch zwei Magnetventile abgeschlossenes Kammervolumen zum Abmessen einer bestimmten Urirunenge verwendet wird und im zweiten Fall ein Meßvolumen an Urin zwischen zwei Rollen einer Schlauchpumpe abgemessen wird. Die Anzahl der abgemessenen Kammervolumen wird dabei zur Ermittlung des Gesamtvolumens an gesammeltem Urin verwendet. Bei beiden bekannten Vorrichtungen ist nachteilig, daß das Betätigen der Magnetventile bzw. das Betreiben der Schlauchpumpe einen relativ hohen Energieaufwand nach sich zieht, der darüber hinaus kontinuierlich erbracht werden muß. Aus diesem Grunde ist es bei den zuletzt beschriebenen Prinzipien nicht möglich, ein automatisches Urinmeßgerät zu konstruieren, das ohne eine Energieversorgung mittels eines Netzkabels auskommt. Es hat sich aber herausgestellt, daß die Anzahl an Verkabelungen an einem Krankenbett einer Internsivstation beim heutigen Stande der Technik bereits so groß ist, daß Intensivpflegepersonal und Ärzte einem Gerät den Vorzug geben, das unabhängig arbeitet, d.h. ohne ein Netzkabel betrieben werden kann. Dabei ist es unter dem Gesichtspunkt einer Betriebsvereinfachung insbesondere wünschenswert, daß ein ohne Netzkabel betriebenes Gerät eine Betriebsdauer aufweist, die einer durchschnittlichen Liegedauer eines Intensivpatienten entspricht. Wünschenswert ist ein Betriebszeitraum des Gerätes über zumindest zwei Wochen, ohne daß eine Akkumulatorzelle o.ä. getauscht werden muß.

Weiterhin ist aus der US-A-4,745,929 ein Gerät bekannt, bei dem der Flüssigkeitsstand einer Urinsäule mittels einer Reihe von Lichtschranken überwacht wird, die übereinander gestaffelt angeordnet sind. An diesem Gerät ist ebenfalls wie bereits bei anderen geschilderten Geräten nachteilig, daß mittels Elektromagneten betätigte Ventilmittel notwendig sind, die einen hohen Stromverbrauch bedingen, und dementsprechend eine Konstruktion des Urinmeßgerätes ohne ständige Stromversorgung mittels eines Netzkabels unmöglich machen.

Aus der EP-A-0 073 156 ist eine weitere Vorrichtung zum Messen eines Urinfluses bekannt, die ebenfalls mittels eines optischen Sensors die Füllung einer Meßkammer überwacht und bei der daraufhin eine Pumpe eingeschaltet wird. Wie auch bei den zuvor beschriebenen Vorrichtungen ist hier nachteilig, daß der konstruktive Aufwand und Stromverbrauch zu hoch ist, um ein Meßgerät zu konstruieren, das dem Betriebsalltag auf einer Intensivstation Rechnung trägt.

Die eingangs erwähnte DE-A-35 44 031 wird als gattungsgemäß angesehen. Diese Druckschrift beschreibt eine Vorrichtung zur Messung des Gewichtes von Flüssigkeit (Urin). Ein Computer ermittelt aus den elektrisch umgesetzten Signalen das spezifische Gewicht, die Temperatur, das Volumen und die Zeit. Problematisch allerdings wird es, wenn das Gehäuse oder dessen Halterung nicht exakt senkrecht herabhängt, da dann nämlich die Gewichtskraftmessung mit Fehlern behaftet ist.

Die US-A-4,712,567 beschreibt ein Meßgerät zum automatischen Überwachen und Erfassen des Urinvolumens und durchschnittlichen Urinvolumens eines katheterisierten Patienten. Der Sammelbehälter des bekannten Meßgerätes ist an einer Basisstation aufgehängt, die über einen Neigungssensor verfügt, der bei einer bestimmten Schräglage die Messung unterbricht. Eine Korrektur der Meßwerte erfolgt bei dem bekannten Meßgeräte hingegen nicht.

Aus der EP-A-0 235 386 ist ein Meßbehälter für einen Tankwagen bekannt, der auf einer Stützfläche schräggestellt ist, so daß dieser keinerlei das Meßergebnis verfälschende Krafteinwirkungen erfährt. Zur Erfassung der Schräglage des Behälters sind zwei Meßeinrichtungen vorgesehen, die in zwei im rechten Winkel zueinander stehenden Ebenen arbeiten.

Der Erfindung liegt die Aufgabe zugrunde, ein gattungsgemäßes Urinmeßgerät so weiterzubilden, daß eine Schieflage seines Gehäuses bzw. der Halterung des Urinmeßgerätes und damit eine Fehlmessung der Gewichtskraft kompensiert wird und gleichzeitig die Dichte des Urins ermittelt wird.

Die Lösung dieser Aufgabe erfolgt erfindungsgemäß mit den im Patentanspruch 1 angegebenen Merkmalen.

Um zusätzlich zum Uringesamtvolumen und zum durchschnittlichen Volumenfluß in einem bestimmten Zeitintervall die Dichte des Urins mit einer Genauigkeit zu ermitteln, die zumindest der Genauigkeit der bisher verwendeten chemischen Teststäbchen entspricht, ist vorgesehen, daß die elektrische Leitfähigkeit des Urins gemessen wird, vorzugsweise mittels einer an sich bekannten Vier-Elektrodenanordnung, und daß aus der Leiffähigkeit über einen in Speichern der Recheneinheit abgelegten funktionalen Zusammenhang auf die spezifische Dichte des Urins geschlossen wird. Der Erfindung liegt hier die überraschende Erkenntnis zugrunde, daß die spezifische Dichte im Rahmen der Genauigkeit, wie sie in der Medizin für diagnostische Zwecke gefordert wird, nahezu ausschließlich durch den Anteil an Elektrolyten beeinflußt wird, während die im Urin enthaltenen Nicht-Elektrolyten, wie beispielsweise Harnstoff und Glukose, Leitfähigkeit und Dichte nur in untergeordnetem Maße beeinflussen.

Der Erfindung liegt dabei unter anderem die Erkenntnis zugrunde, daß ein elektrischer Kraftaufnehmer mit sehr geringer Hilfsenergie betrieben werden kann, beispielsweise in Form eines Dehnungsmeßstreifens (DMS).

Weiterhin macht die Erfindung von der Erkenntnis Gebrauch, daß die beiden Werte, die zusätzlich zur Bestimmung des Urinvolumens aus der Gewichtskraft der Urinmenge benötigt werden, nur innerhalb so geringer Grenzen schwanken, daß sie für diesen Zweck im Rahmen der Genauigkeit, die im medizinischen Alltag gefordert werden müssen, als Konstanten angenommen werden können. Die erste dieser Einflußgrößen ist die Erdbeschleunigung, die geringfügig in Abhängigkeit von der geographischen Breite schwankt. Der zweite Wert ist die Dichte. Die mit der Dichte von destilliertem Wasser normierte Dichte schwankt beim menschlichen Urin zwischen 1,001 bis 1,035. Dieser relative Fehler führt bei der Ermittlung des Urinvolumens in der geschilderten Weise zu einem relativen Fehler, der kleiner ist als 2 %, oder aber bei geringen Mengen (0-100 ml) kleiner ist als 2 ml. Der so erhaltene Wert ist damit kleiner als die Genauigkeit, die sich mit der herkömmlichen Skalierung von auf dem Markt befindlichen Urinmeßbehältern erreichen läßt.

Erfindungsgemäß kann weiterhin vorgesehen sein, daß die oben diskutierten Werte für mittlere Erdbeschleunigung und ermittelte Dichte menschlichen Urins zu einem Wert, d.h. der mittleren Wichte von Urin, kombiniert sind.

Weiterhin ist erfindungsgemäß vorgesehen, daß das Urinmeßgerät einen Zeitgeber aufweist, so daß die Volumenzunahme im Urinsammelbeutel nach Ablauf von bestimmten Zeitintervallen festgestellt werden kann. Indem die Rechenmittel dazu benutzt werden, die ermittelte Volumenzunahme des Urins in dem Zeitintervall durch die Länge des Zeitintervalls zu dividieren, wird ein durchschnittlicher Volumenfluß für ein bestimmtes Zeitintervall ermittelt. Vorteilhaft werden die ermittelten Werte für Gesamtvolumen und durchschnittlichen Volumenfluß in den einzelnen Zeitintervallen in einer alphanumerischen Anzeige angezeigt und in entsprechenden Speicherplätzen für eine spätere Verwendung abgelegt. Weiterhin kann vorgesehen sein, die ermittelten Kennwerte über eine Druckerschnittstelle auszudrucken und so ein Protokoll anzufertigen.

Weiterhin ist insbesondere vorgesehen, daß der erwähnte Zeittaktgeber mit einer Stromsparschaltung kombiniert ist, die die wesentlichen Stromverbraucher des Urinmeßgerätes nur zur jeweiligen tatsächlichen Messung am Intervallende einschaltet. Auf diese Weise wird der Stromverbrauch weiter reduziert, so daß in Verbindung mit den bereits stromsparend ausgelegten Sensormitteln ein so geringer Stromverbrauch des Gesamtgerätes erreicht wird, daß eine Stromversorgung aus eingebauten Akkumulatorzellen (Akkus) ermöglicht wird. Auf diese Weise kann auf ein Netzkabel verzichtet werden, und es wird ein autonom arbeitendes Gerät ermöglicht, das beispielsweise an das Krankenbett angehängt zusammen mit dem Patienten verfahren werden kann, ohne daß eine Diskonnektierung von Netzkabeln o.ä. notwendig ist.

Die erfindungsgemäße Ausgestaltung eines Urinmeßgerätes ermöglicht jedoch nicht nur einen stromsparenden Betrieb des Urinmeßgerätes und damit eine netzunabhängige Stromversorgung, sondern ermöglicht es auch, das Urinsammelgefäß in Form eines Kunststoffbeutels auszuführen, der mit einem zu dem Katheter führenden Kunststoffschlauch einstückig verbunden ist und als Wegwerf- bzw. Einwegteil billig produziert werden kann. Dabei kann insbesondere vorgesehen sein, in dem Abflußschlauch eine Tropfkammer vorzusehen, wie sie aus dem Stand der Technik bekannt ist, um eine retrograde Verkeimung des gesamten Systems zu unterbinden. Diese Tropfkammer kann ebenfalls aus Kunststoff gefertigt und einteilig mit den beiden anderen Komponenten, d.h. dem Kunststoffbeutel als Urinsammelgefäß und dem Abflußschlauch verbunden sein. Auf diese Weise wird ein geschlossenes System geschaffen, das hohe Sicherheit gegen Verkeimung bietet, das billig als Einwegteil herzustellen ist und das trotzdem eine genaue automatische Erfassung der erläuterten Kennwerte des Urins ermöglicht.

Für die Ermittlung der Leitfähigkeit des Urins kann eine integrierbare Leitfähigkeitsmeßvorrichtung mit vier Elektroden verwendet werden, wie sie beispielsweise aus der DE-OS 41 13 033 bekannt ist.

Vorzugsweise wird die aus vier Elektroden bestehende Meßanordnung für die Leitfähigkeit in die Tropfkammer integriert. Um stets ein ausreichendes, die Elektroden benetzendes Meßvolumen an Urin für die Leitfähigkeitsmessung zur Verfügung zu haben, das stetig ausgetauscht wird, so daß tatsächlich der neue, mittlerweile nachgetropfte Urin auf seine Leitfähigkeit hin gemessen wird, ist erfindungsgemäß bevorzugt vorgesehen, daß die Tropfkammer einen U-förmigen Kanalabschnitt aufweist, dessen einer senkrecht angeordneter Schenkel in der Tropfenfall-Linie der Tropfkammer angeordnet ist, und dessen anderer Schenkel zu einem Überlaufraum führt. Dabei ist bevorzugt vorgesehen, daß der U-förmige Kanal in der Tropfkammer zumindest teilweise durch Zwischenwände begrenzt wird, wobei die vier Elektroden vorzugsweise in Form von Blechstreifen ausgeführt sind und in eine dieser Zwischenwände eingeschweißt sind. Vorzugsweise ragen die Blechelektroden in Form von federnden Zungen über die rückwärtige Wand der Tropfkammer hinaus und dienen der Kontaktierung mit entsprechenden Kontaktpunkten, die mit dem Meßgerät verbunden sind.

Vorzugsweise ist weiterhin vorgesehen, daß die Tropfkammer aus weißem Kunststoff hergestellt ist, und daß die vordere Wand der Tropfkammer aus einem durchsichtigen Kunststoff besteht. Auf diese Weise läßt sich das in dem U-förmigen Kanal stehende Urinvolumen auch optisch einfach kontrollieren, ohne daß sich der diagnostizierende Arzt übermäßig bücken muß oder durch einen andersfarbigen Hintergrund, der durch den zu beobachtenden Urin hindurchscheint, irritiert wird.

Weiterhin ist vorzugsweise vorgesehen, daß die Tropfkammer in das Meßgerät einsetzbar ist und an diesem fixiert wird, so daß die Tropfkammer als Zugentlastung dient und auf diese Weise sicherstellt, daß über einen zwischen der Tropfkammer und dem Urinsammelbeutel angeordneten Verbindungsschlauchabschnitt keine das Meßergebnis des Kraftaufnehmers verfälschenden Kräfte eingeleitet werden. Dazu ist bevorzugt vorgesehen, daß das Urinmeßgerät ein Gehäuse aufweist, in dem ein senkrecht angeordneter Befestigungsschacht in Form einer kanalartigen Einbuchtung angeordnet ist, in der die Tropfkammer verriegelbar ist. Auf diese Weise wird einerseits eine Zugentlastung geschaffen, andererseits ist die aus Plastik bestehende Tropfkammer in dem Befestigungsschacht mit der Oberfläche des Gehäuses bündig abschließend angeordnet, so daß einerseits ein gefälliges Aussehen erzielt, andererseits die Tropfkammer vor versehentlichen Beschädigungen geschützt wird.

Bevorzugt ist vorgesehen, daß auf der Rückseite der Tropfkammer elastisch federnde Zungen ausgebildet sind, die einen in dem Befestigungsschacht des Gehäuses angeordneten Befestigungsvorsprung einrastend umgreifen. Diese Ausführungsform bietet den Vorteil, daß eine Befestigungsmöglichkeit geschaffen wird, die sich zusammen mit der Tropfkammer in großen Stückzahlen billig herstellen läßt. Vorzugsweise sind in dem Gehäuse des Urinmeßgerätes neben dem senkrecht angeordneten Befestigungsschacht rechts und links der eingelegten Tropfkammer Fingergriffmulden vorgesehen, die es erlauben, eine eingelegte Tropfkammer mit Daumen und Zeigefinger zu ergreifen und aus dem Urinmeßgerät herauszuziehen.

Um den erfindungsgemäß aus der Leitfähigkeit des Urins ermittelten Wert für die Dichte des Urins weiter zu korrigieren, kann vorgesehen sein, die Temperatur des in der Tropfkammer stehenden Urinvolumens zu messen. Hierzu ist bevorzugt vorgesehen, daß zumindest einer der elektrischen Kontaktpunkte des Befestigungssockels im Befestigungsschacht des Gehäuses gut wärmeleitend ausgebildet ist und mit einem Tempratursensor verbunden ist. Dieser kann beispielsweise ein NTC- oder PTC-Widerstand sein. Die Temperatur des elektrischen Kontaktpunktes und damit des Temperatursensors wird bei einer gut wärmeleitenden Ausbildung nach einiger Zeit der Temperatur der federnd auf ihm lastenden Zunge bzw. der zugehörigen Blechelektrode entsprechen, die von dem in der Tropfkammer sich befindenden Urin umspült wird.

Dabei ist zu betonen, daß auch bei dieser Meßwerterfassung der erfindungsgemäßen Aufgabe Rechnung getragen wird, das unmittelbar mit Urin in Kontakt kommende Bauteil möglichst einfach und kostengünstig auszuführen. Insbesondere wird erfindungsgemäß die Anzahl überhaupt zu fertigender Teile reduziert, da einer der vier Elektroden neben einer elektrischen Leitung darüber hinaus die Aufgabe eines Wärmeleiters zugewiesen wird.

Vorteilhaft kann eine Vorrichtung zum Messen der Körpertemperatur des katheterisierten Patienten in das erfindungsgemäße Urinmeßgerät dadurch integeriert werden, daß in die Spitze des Katheters ein Temperaturmeßelement, beispielsweise in Form eines NTC- oder PTC-Widerstandes integriert ist, das über eine elektrische Leitung, die gegebenenfalls auch in den Abflußschlauch integriert sein kann, mit dem Urinmeßgerät verbunden ist.

Das hier beschriebene Meßgerät mit den erwähnten Merkmalen ist in erster Linie für die Erfassung von Urinkennwerten gedacht, jedoch ist eine Verwendung für andere Körperflüssigkeiten nicht ausgeschlossen.

Die Erfindung wird im folgenden anhand eines in der Zeichnung dargestellten Ausführungsbeispieles näher erläutert. In der Zeichnung zeigen:
- **Fig. 1**: eine Ansicht eines erfindungsgemäßen Urinmeßgerätes mit eingesetzter Tropfkammer und angehängtem Urinsammelbeutel,
- **Fig. 2**: einen Schnitt durch die in Fig. 1 dargestellte Anordnung,
- **Fig. 3**: eine Ansicht der in Fig. 1 dargestellten Tropfkammer in vergrößertem Maßstab,
- **Fig. 4**: einen Schnitt längs der Linie IV-IV in Fig. 3,
- **Fig. 5**: einen Schnitt durch die Tropfkammer längs der Linie V-V in Fig. 3,
- **Fig. 6**: ein Blockschaltbild der elektrischen Komponenten des Urinmeßgerätes, und
- **Fig. 7**: eine schematische Darstellung des Zusammenhangs zwischen relativer Dichte und elektrischer Leitfähigkeit von menschlichem Urin, gemessen mit der Genauigkeit von herkömmlichen Teststäbchen.

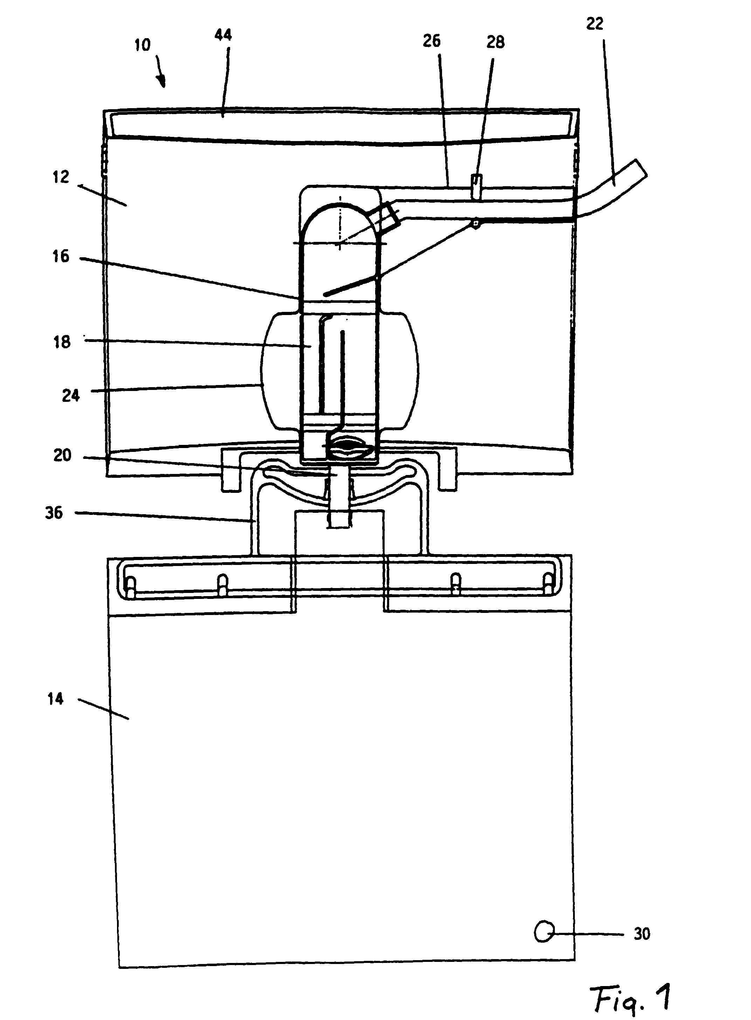

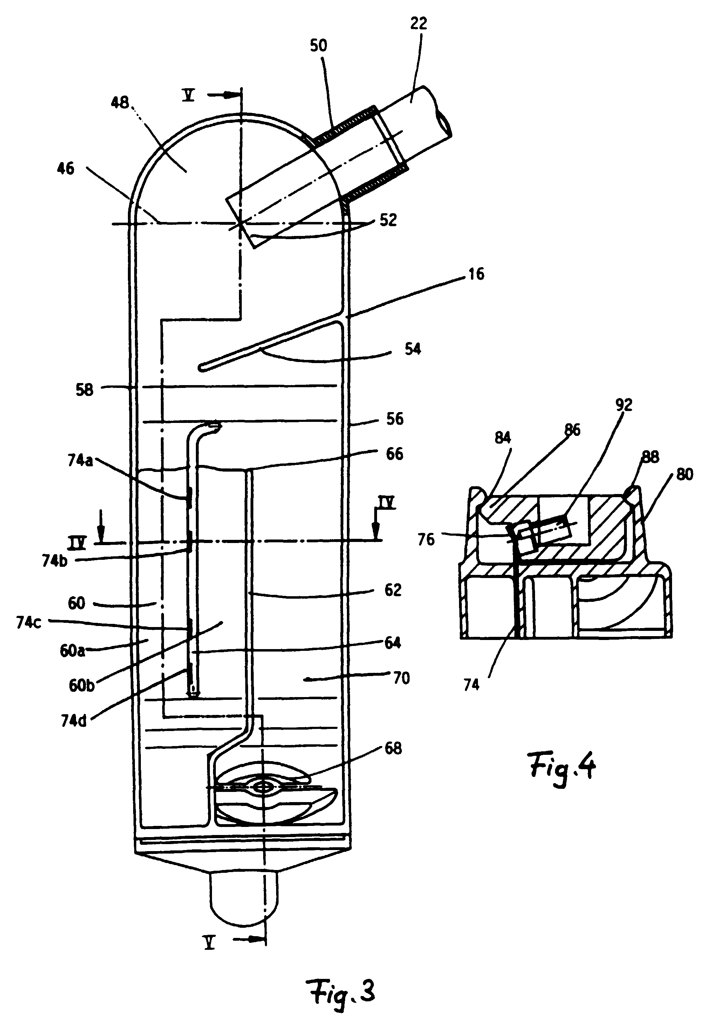

Das in Fig. 1 dargestellte Urinmeßgerät 10 weist ein Gehäuse 12 auf, das mittels einer nicht dargestellten Befestigungseinrichtung vorzugsweise an einer Längs- oder Querstrebe eines Krankenbettes so befestigt wird, daß ein Abstand zum Fußboden verbleibt, der es zuläßt, einen Urinsammelbeutel 14 freihängend unter dem Gehäuse 12 anzuordnen, ohne daß dieser auf dem Boden aufliegt. Das Gehäuse 12 weist einen Befestigungsschacht 16 auf, in dem eine auswechselbare Tropfkammer 18 befestigt ist. Die Tropfkammer kann auf verschiedene Arten in dem Befestigungsschacht gehalten werden, beispielsweise magnetisch oder durch formschlüssige Verriegelungsmittel, wie sie anhand der Fig. 3 bis 5 noch näher erläutert werden.

Am unteren Ende der Tropfkammer 18 schließt sich ein Schlauchabschnitt 20 an, der die Tropfkammer mit dem Urinsammelbeutel 14 verbindet. Am oberen Ende der Tropfkammer 16 schließt sich ein Abflußschlauch 22 an, dcr zu dem in der Blase des Patienten angeordneten, nicht dargestellten Katheter führt. Der durch den Katheter aus der Blase des Patienten abgeleitete Urin fließt - da das am Bettrahmen befestigte Urinmeßgerät tiefer liegt als der Patient - unter dem Einfluß der Schwerkraft durch den Abflußschlauch 22 in die Tropfkammer 18, von dort durch den Schlauchabschnitt 20 und wird in dem Urinsammelbeutel 14 gesammelt. Abflußschlauch 22, Tropfkammer 18, Verbindungsschlauchabschnitt 20 und Urinsammelbeutel 14 sind aus Kunststoff hergestellt und miteinander verschweißt. Sie bilden zusammen ein Wegwerfteil, das nach einer Betriebszeit von etwa zwei Wochen oder einem Patientenwechsel weggeworfen oder einer Kunststoffwiederverwertung zugeführt werden kann.

Der Befestigungsschacht 16 des Gehäuses 12 erweitert sich rechts und links der Tropfkammer 18 über eine bestimmte Höhe des Befestigungsschachtes zu Fingergriffmulden 24, die ein Herausnehmen der Tropfkammer ermöglichen. Am oberen Ende des Befestigungsschachtes 16 schließt sich im wesentlichen rechtwinklig ein schmalerer Aufnahmekanal 26 für den Abflußschlauch 22 an. Im Aufnahmekanal 26 sind Klemm-Mittel 28 angeordnet, mit denen der Abflußschlauch 22 fixiert werden kann.

Der Urinsammelbeutel 14 kann in einer seiner beiden unteren Eckbereiche ein Abflußventil 30 aufweisen, mit dem eine Entleerung ermöglicht wird. Der Urinsammelbeutel hat vorzugsweise ein Volumen zwischen zwei und vier Litern und kann somit den durchschnittlichen 24-Stunden-Harn eines Patienten aufnehmen.

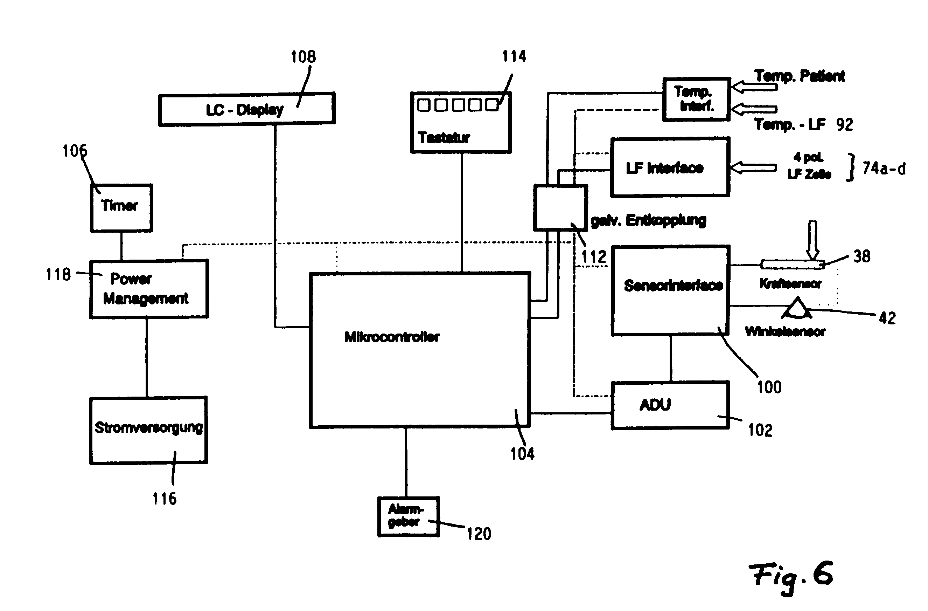

An seiner oberen Kante 32 ist der vorzugsweise im Extrudierverfahren hergestellte Kunststoffbeutel 14 mit einer Plastik-Querleiste 34 verstärkt, die einen U-förmigen Querschnitt aufweist. Die Querleiste 34 geht einstückig in ein bügelförmiges Haltemittel 36 über, das kraftschlüssig mit cinem Kraftaufnehmer 38 verbindbar ist. Beim vorliegenden Ausführungsbeispiel wird das bügelförmige Haltemittel 36 in einen mit dem Kraftaufnehmer 38 verbundenen Haken eingehängt. Der Kraftaufnehmer 38 ist mit einer Halterung verbunden, die mit dem Bettrahmen verbunden ist. Bei dem vorliegenden Ausführungsbeispiel sind Halterung und ein Gehäuse 12 in Integralbauweise ausgeführt. Der Kraftaufnehmer 38 kann beispielsweise in Form eines Dehnungsmeßstreifens (DMS) ausgeführt sein. Mittels des Kraftaufnehmers 38 kann die von der Masse des Kunststoffbeutels 14, der Verstärkungsleiste 34, des Haltemittels 36 und des im Beutel 14 gesammelten Urins 40 in das Gehäuse bzw. die Halterung 12 eingeleitete Gewichtskraft gemessen werden, vorausgesetzt, daß die Wirkungslinie des Kraftaufnehmers 38 senkrecht ausgerichtet ist. Um Abweichungen der Wirkungslinie des Kraftaufnehmers 38 aus der Senkrechten zu kompensieren, sind zwei in zwei senkrecht aufeinander stehenden Ebenen angeordnete Winkelsensoren 42 (Fig. 6) vorgesehen, mittels derer eine Fehlstellung des gesamten Gehäuses 12 und damit des Kraftaufnehmers 38 kompensiert werden kann, indem das von dem Kraftaufnehmer 38 gelieferte Signal durch Multiplikation mit dem Cosinus des jeweiligen Fehlwinkels korrigiert wird.

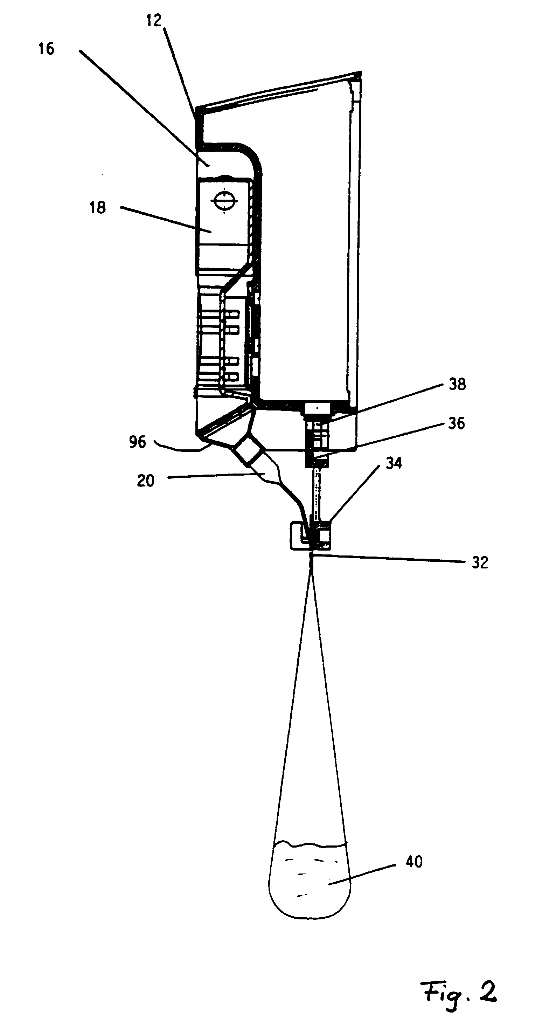

Wie aus der Zusammenschau der Fig. 1 und 2 ersichtlich, übernimmt die in dem Befestigungsschacht 16 verriegelte Tropfkammer 18 die Funktion einer Zugentlastung. Auf den Abflußschlauch 22 wirkende Kräfte - beispielsweise wegen einer Bewegung des Patienten - führen nicht zu einer Krafteinleitung in den Verbindungsschlauchabschnitt 20, so daß das Gewicht des Sammelbeutels 14 nicht scheinbar veringert werden kann. Weiterhin wird durch eine entsprechende Überlänge des Verbindungsschlauchabschnitts 20 sichergestellt, daß das Gesamtgewicht des Sammelbeutels 14 über die Halterung 36 ausschließlich in den Kraftaufnehmer 38 eingeleitet wird, ohne daß am Verbindungsschlauchabschnitt 20 zusätzliche Lagerkräfte erzeugt werden, die das Ergebnis verfälschten.

Die ermittelten Werte für das Gesamtvolumen 40 des gesammmelten Urins, sowie für errechnete, durchschnittliche Urinvolumenflüsse in einzelnen Zeitintervallen, gesamte Meßzeit, etc. werden in einer alphanumerischen Anzeige 44 angezeigt. Die alphanumerische Anzeige 44 kann in an sich bekannter Weise aus einer LCD-Punktmatrix bestehen o.ä..

Die Fig. 3 bis 5 zeigen die auswechselbare Tropfkammer 18 des Urinmeßgerätes in vergrößertem Maßstab. Die Tropfkammer 18 vereinigt mehrere Funktionen in sich. Zunächst wird durch die freie Fallstrecke der Urintropfen eine retrograde Verkeimung des Abflußschlauches 22 und damit eine Infektion des Patienten unterbunden, wie dies auch bei anderen aus dem Stand der Technik bekannten Urinmeßgeräten üblich ist. Die geometrische Grundform der Tropfkammer entspricht im wesentlichen der eines Quaders, wobei im Bereich der imaginären oberen Standfläche 46 eine Kreiszylinderhälfte 48 aufgesetzt ist. An den kreiszylinderförmigen Abschnitt 48 schließt sich ein Stutzen 50 an, in den der Abflußschlauch 22 eingeschweißt ist. Der Abflußschlauch 22 ragt über ein gewisses Maß in die Tropfkammer hinein, etwa bis zur Mittellinie der im wesentlichen quaderförmigen Tropfkammer. Unterhalb der Öffnung 52 des Schlauches 22 ist eine unter einem Winkel zur Waagerechten angeordnete Prallplatte 54 angeordnet, die zu einer Seite hin mit einer Seitenwand 56 der Tropfkammer verbunden ist. Die Prallplatte 54 endet mit einem gewissen Abstand vor der gegenüberliegenden Seitenwand 58 der Tropfkammer, so daß der aus der Öffnung 52 des Abflußschlauches 22 tropfende Urin auf die Prallplatte 54 tropft und dann aufgrund der Neigung derselben abfließt. Der Urin fließt von der Prallplatte 54 und fällt in einem U-förmigen Kanal 60, der durch die Seitenwand 58 der Tropfkammer sowie durch eine erste Zwischenwand 62 begrenzt wird. Die beiden Schenkel 60a und 60b des U-förmigen Kanals werden durch eine zweite Zwischenwand 64 voneinander getrennt. Der von der Prallplatte 54 herabtropfende Urin füllt den U-förmigen Kanal 60 nach und nach auf, wobei sich aufgrund des hydrostatischen Gleichgewichts ein gleicher Füllstand in beiden Schenkeln ausbildet, bis der Urinspiegel die obere Kante 66 der ersten Zwischenwand 62 erreicht. Weiterer von der Prallplatte 54 in den ersten Schenkel 60a des U-förmigen Kanals tropfender Urin führt aufgrund des hydrostatischen Gleichgewichts dazu, daß ein entsprechendes Volumen an Urin über die obere Kante 66 der ersten Zwischenwand 62 fließt und über ein Rückschlagventil 68 in den Verbindungsschlauch 20 gelangt, von wo aus es in den Sammelbeutel 14 abfließt. Das Rückschlagventil 68 kann federvorbelastet sein, so daß es erst dann öffnet, wenn die Höhe des Urinspiegels in der durch die erste Seitenwand 56 und die erste Zwischenwand 62 definierten Überlaufkammer 70 einen bestimmten Wert übersteigt. Durch die Anordnung des U-förmigen Kanals wird eine Siphonwirkung erreicht, d.h. es steht stets ein bestimmtes Flüssigkeitsvolumen an Urin in der Tropfkammer, dessen Stoffmenge stetig ausgetauscht wird. Das im wesentlichn quaderförmige Tropfkammerteil, bestehend aus den beiden Seitenwänden 56 und 58, einer Rückwand 59, erster Zwischenwand 62, zweiter Zwischenwand 64 und aufgesetzter Kreiszylinderhälfte 48, ist vorzugsweise aus weißgefärbtem Kunststoff gefertigt, während eine die Tropfkammer zur Betrachterseite hin verschließende Frontseite 72 aus durchsichtigem Kunststoff gefertigt ist. Auf diese Weise steht dem beobachtenden Arzt oder Intensivpfleger ständig ein Urinvolumen zur Sichtkontrolle zur Verfügung, dessen Farbe durch die durchsichtige Frontseite 72 vor dem weißfarbigen Hintergrund begutachtet werden kann.

Zur Messung der Leitfähigkeit des Urins sind in der Zwischenwand 64 vier Elektroden 74a, 74b, 74c, 74d in Form von Blechstreifen eingelassen, deren rückwärtiger Teil über die Rückwand 59 der Tropfkammer hinaus verlängert ist und federnde Kontaktzungen 76a bis 76d bildet. Die vier Elektroden dienen der Leitfähigkeitsmessung des in dem U-förmigen Kanal 60 stehenden Urins mittels der bekannten Vier-Elektroden-Meßmethode. Den beiden äußeren Elektroden 74a und 74d wird mittels einer Konstantstromquelle ein Strom definierter Größe aufgeprägt, der den Urin auf der zwischen den beiden genannten Elektroden liegenden Strecke durchfließt. Die beiden inneren Elektroden 74b und 74c bilden eine Abgriffsmöglichkeit für eine Potentialdifferenz, die der Leitfähigkeit des Urins umgekehrt proportional ist. Durch die Vier-Elektrodenanordnung werden die Einflüsse von Übergangswiderständen zwischen Elektroden und Urin einerseits und zwischen Kontaktzungen 76a bis 76d und ihnen zugeordneten Kontaktpunkten andererseits eliminiert, so daß die Tropfkammer über ihre gesamte Lebensdauer von ca. zwei Wochen ohne Nacheichen zur Leitfähigkeitsmessung eingesetzt werden kann.

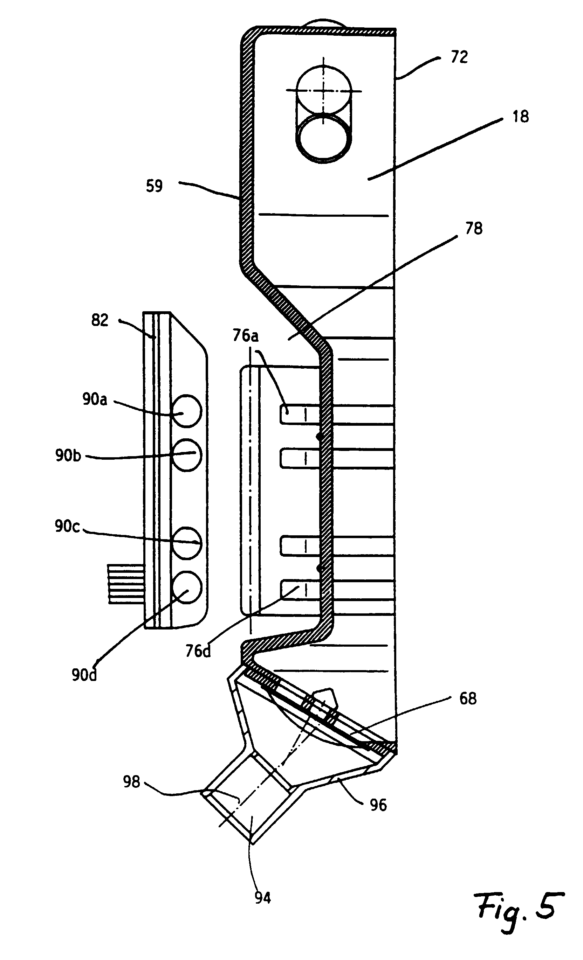

Wie aus Fig. 5 ersichtlich, weist die Rückwand 59 der Tropfkammer 18 eine Einbuchtung 78 auf, in der zwei federnde Zungen 80 angeordnet sind. In dem Befestigungsschacht 16 des Meßgerätes ist ein im wesentlichen komplementär zu der Ausbuchtung 78 geformter Befestigungssockel 82 angeordnet, der zwei Hinterschneidungen 84 aufweisende Kantenbereiche 86 hat. Die federnden Zungen 80 werden durch die Kanten 86 beim Einsetzen der Tropfkammer 18 in den Befestigungsschacht 16 aufgepreizt und rasten beim weiteren Eindrücken der Tropfkammer in dem Befestigungsschacht mit entsprechend ausgebildeten Nasen 88 hinter den Hinterschneidungen 84 ein. Gleichzeitig kommen die vier Kontaktzungen 76a bis 76d, die auf der Rückseite der Tropfkammer 18 zwischen den federnden Zungen 80 angeordnet sind, mit entsprechend ausgebildeten Kontaktpunkten 90a bis 90d in federnden Kontakt und schaffen so eine elektrische Verbindung zwischen jeweils einer Elektrode 74a bis 74d und dem elektrischen Teil des Urinmeßgerätes.

Wie Fig. 4 zeigt, kann zumindest einer der Kontaktpunkte 90a bis 90d in Form einer hohlgebohrten Schraube 92 ausgeführt sein, die vorzugsweise aus einem gut wärmeleitenden Material besteht. In der Bohrung ist ein - nicht dargestelltes - Temperaturmeßelement, beispielsweise in Form eines PTC- oder NTC-Widerstandes wärmeleitend eingeklebt. Auf diese Weise wird eine Meßmöglichkeit für die Temperatur des in dem U-förmigen Kanal 60 stehenden Urins geschaffen, d.h. die Temperatur wird an der gleichen Stelle bestimmt, an der die Leitfähigkeit des Urins erfaßt wird. Das von dem Temperaturmeßelement in der Hohlschraube 92 gelieferte Meßsignal und die daraus ermittelte Temperatur kann zur Korrektur des aus der Leitfähigkeit ermittelten Wertes für die Dichte des in der Kammer stehenden Urins benutzt werden.

Wie Fig. 5 zeigt, ist am unteren Ende der Tropfkammer ein Anschlußstutzen 94 für den Verbindungsschlauchabschnitt 20 vorgesehen, der mittels eines trichterförmig sich erweiterten Zwischenbauteils 96 im Bereich des Rückschlagventils 68 mit der Tropfkammer verbunden ist. Dabei ist der Anschlußquerschnitt zwischen dem trichterförmigen Bauteil 96 und der eigentlichen Tropfkammer 18 unter einem Winkel von ca. 30° angestellt, so daß die Mittelachse 98 des Stutzens 94 in bezug auf die Frontseite 72 der Tropfkammer 18 nach hinten gerichtet ist. Auf diese Weise wird - wie aus Fig. 2 ersichtlich - eine im wesentlichen den Außenkanten des Meßgerätes 12 folgende, kompakte Schlauchführung erreicht sowie ein Übergang zum Befestigungsbereich des Urinsammelbeutels 14 geschaffen.

Fig. 6 zeigt den prinzipiellen elektrischen Aufbau des erfindungsgemäßen Urinmeßgerätes. Die von dem Kraftsensor 38 und den beiden Winkelsensoren 42 gelieferten Signale werden in einer Sensorschnittstelle 100 aufgenommen und an einen Analog-Digital-Umsetzer 102 geleitet. Die digital codierten Signale werden weiter zu einer zentralen Recheneinheit 104 geleitet, die über integrierte, nicht näher dargestellte Speichermittel verfügt. In dem auch als Microcontroller bezeichneten Bauteil 104, d.h. in der Recheneinheit, wird zunächst das vom Kraftsensor gelieferte Signal mittels der von den beiden Winkelsensoren gelieferten Signale korrigiert. Ist die Winkelabweichung Null, so ist der zugehörige Cosinus des Fehlwinkels gleich 1, d.h. das Signal bleibt unverändert.

Von dem so ermittelten Wert für die Gewichtskraft der gesamten an dem Kraftsensor über die Haltevorrichtung 36 hängenden Masse wird zunächst die bekannte Gewichtskraft des Urinsammelbeutels 14, d.h. die gesamte Tara, subtrahiert. Der so erhaltene Wert repräsentiert die Gewichtskraft des in dem Urinsammelbeutel gesammelten Urins 40. Der so erhaltene Wert wird durch einen gespeicherten Wert für die mittlere Erdbeschleunigung dividiert. Der so erhaltene Wert entspricht der Masse des gesammelten Urins. Dieser Wert wird durch einen Wert für die Dichte des Urins dividiert und führt so zu einem Gesamtvolumen des gesammelten Urins 40.

Ein Zeittaktgeber 106 mißt die Zeit und das Gesamtvolumen des angesammelten Urins 40 wird in der zuvor beschriebenen Weise in bestimmten Zeitabständen, beispielsweise drei Minuten, ermittelt. Aus der Differenz zweier gespeicherter Volumina kann der Volumenzuwachs innerhalb eines bestimmten Zeitintervalls bestimmt werden. Der Quotient aus dem ermittelten Volumenzuwachs und dem Zeitintervall ergibt den durchschnittlichen Urinvolumenfluß für das jeweilige Zeitintervall. Die jeweiligen Werte für Gesamtvolumen und durchschnittlichem Volumenfluß für jeweils ein Zeitintervall werden in einem Speicher abgelegt und können in einer Anzeigeeinheit 108 alphanumerisch angezeigt werden.

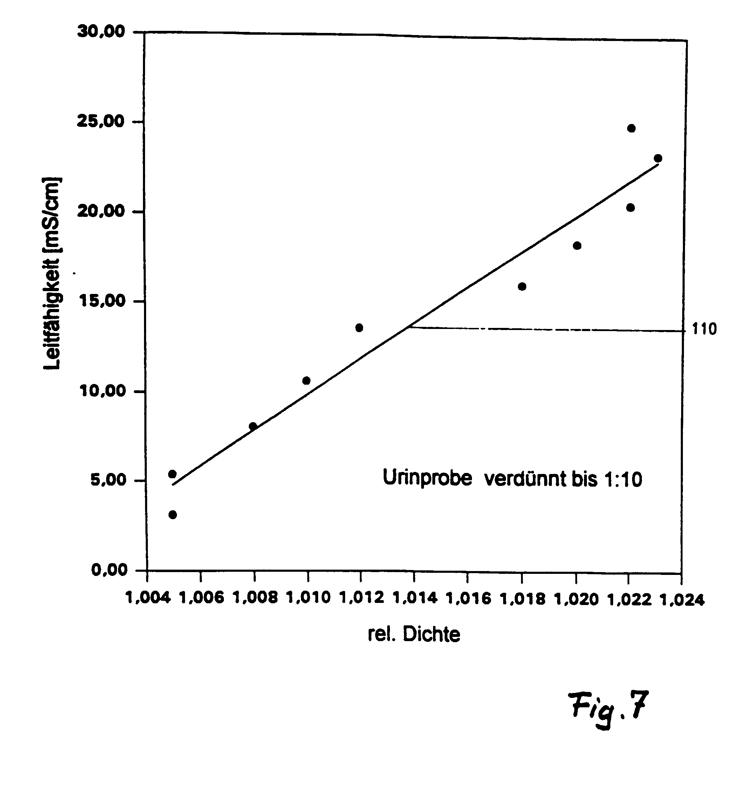

Der Ermittlung der Dichte dient wie zuvor beschrieben die Vier-Elektrodenanordnung 74a bis 74d in der Tropfkammer 18. In an sich bekannter Weise wird die Leitfähigkeit des Urins bestimmt. Sodann wird im Microcontroller auf eine tabellenartig abgelegte Funktion zurückgegriffen, die in Fig. 7 dargestellt ist.

Fig. 7 zeigt in Form von Meßpunkten den Zusammenhang von Leitfähigkeit von menschlichem Urin und dessen relativer Dichte, wie er durch Teststäbchen festgestellt wird, wie sie bisher im Stand der Technik benutzt worden sind. Die Meßstäbchen sind mit einer Substanz beschichtet, die auf die im Urin vorhandenen Elektrolyte anspricht und in einer Folgereaktion einen Farbindikator zum Umschlag bringt. Der durch die Teststäbchen gegebene Zusammenhang wird durch eine Ausgleichsfunktion 110 angenähert und diese Funktion in einem entsprechenden Speicher des Microcontrollers 104 abgelegt. Auf diese Weise kann ein Wert für die relative Dichte des Urins ermittelt und in der Anzeigeeinheit 108 angezeigt werden. Der so ermittelte Wert steht dem Arzt für diagnostische Zwecke zur Verfügung. Er wird darüber hinaus dazu verwendet, die Genauigkeit der Volumenbestimmung des angesammelten Urins dadurch zu steigern, daß anstelle eines Durchschnittswertes für die spezifische Dichte ein entsprechend korrigierter Wert verwendet wird.

Weiterhin ist vorgesehen, daß über das in der Hohlschraube 92 integrierte Temperaturmeßelement ein Signal erzeugt wird, das zu der Temperatur des Urins in der Tropfkammer 18 proportional ist. Der Temperatureinfluß auf die Dichte kann auf diese Weise ebenfalls im Microcontroller durch eine entsprechende Korrekturfunktion korrigiert werden.

Weiterhin ist zusätzlich vorgesehen, an dem Katheter selbst einen temperaturempfindlichen Meßsensor anzubringen, der ein Signal liefert, das ebenfalls über eine Schnittstelle dem Microcontroller zugeführt wird. Das von dem an der Katheterspitze angeordneten Temperaturmeßelement erzeugte Signal wird mit einer entsprechenden Funktion in eine Temperatur umgerechnet und steht dem Überwachungspersonal ebenfalls in einer Anzeige 108 zur Verfügung. Diese zusätzliche Funktion ermöglicht eine kontinuierliche Überwachung der Körpertemperatur des Patienten, ohne daß ein zusätzliches Gerät notwendig wäre. Weiterhin kann vorgesehen sein, daß auch die Körpertemperatur des Patienten jeweils zu den durch den Zeittaktgeber 106 bestimmten Intervallzeitpunkten dokumentiert wird und die in einem entsprechenden Speicher des Microcontrollers 104 abgelegten Meßwerte dokumentiert werden.

Die mit dem Patienten direkt in Berührung kommenden Sensoren, wie der an der Spitze des Katheters angeordnete Temperatursensor oder die mit dem Patienten über eine eventuell mögliche Leitung innerhalb einer Urinsäule indirekt in Berührung kommenden Sensoren, wie die vier Pole der Leitfähigkeitsmeßzelle 74a bis 74d, sind aus Sicherheitsgründen durch eine galvanische Entkopplung 112 von dem Patienten entkoppelt.

Das Gerät weist weiterhin eine Tastatur 114 auf, mit der beispielsweise nach Entleerung des Urinsammelbeutels 14 durch das Entleerungsventil 30 ein neuer Bezugspunkt für den nun folgenden Gewichtszuwachs eingestellt werden kann. Diese Funktion ist insbesondere dann nützlich, wenn - wie beschrieben - der Beutel zwar beispielsweise täglich entleert, aber kontinuierlich weiterverwendet wird, da dann die Tara, d.h. das gesamte an dem Kraftsensor hängende Gewicht, das nicht dem eigentlichen gesammelten Urin zuzurechnen ist, geringfügig größer ist, als das Gewicht des Urinsammelbeutels 14, der Querleiste 32 und des Haltemittels 36, da der Urinsammelbeutel 14 beim Entleeren nicht vollständig entleert wird, sondern noch Urinreste verbleiben, die während eines nachfolgenden Meßzyklus nicht noch einmal erfaßt werden sollen.

Die elektrische Schaltung des erfindungsgemäßen Meßgerätes weist weiterhin eine Akkumulatorzelle 116 auf, die der Stromversorgung des gesamten Gerätes dient. Um den Stromverbrauch zu senken, ist weiterhin ein Stromsparschaltkreis 118 vorgesehen, der von dem Zeittaktgeber 106 angesteuert wird. Durch die Kombination aus Zeittaktgeber 106 und Stromsparschaltkreis 118 wird ein Betrieb des gesamten Gerätes ermöglicht, bei dem lediglich jeweils an den Intervallgrenzen eines Zeitintervalls Messungen vorgenommen werden, so daß der Stromverbruch insgesamt so weit reduziert wird, daß der Einsatz eines Akkus als Stromquelle überhaupt erst ermöglicht wird. Auf diese Weise wird ein Netzkabel entbehrlich, das insbesondere auf Intensivstationen und beim Umbetten des Patienten eine Gefährdungsquelle darstellt.

Schließlich weist die Schaltung gemäß Fig. 6 einen Alarmgeber 120 auf, der vom Microcontroller 104 dann angesteuert wird, wenn die ermittelten Werte, beispielsweise für Urinvolumen, Urinvolumenfluß, Körpertemperatur des Patienten o.ä. außerhalb bestimmter Grenzwerte liegen. Der Alarmgeber 120 kann beispielsweise ein akustisches Signal abgeben, oder aber ein optisches Signal.

Nicht dargestellt in Fig. 6 ist eine Schnittstelle zu einem Drucker o.ä., mit dem ein Protokoll der erfaßten Meßwerte ausgedruckt werden kann.

Obwohl das erfindungsgemäße Urinmeßgerät zum automatischen Überwachen und Erfassen des Urinflusses eines katheterisierten Patienten erfunden worden ist, ist es denkbar, das Gerät zum automatischen Überwachen und Erfassen des Volumenflusses auch anderer Körperflüssigkeiten einzusetzen.

## Patentansprüche

1. Urinmeßgerät zum automatischen Überwachen und Erfassen des Urinflusses eines katheterisierten Patienten, wobei insbesondere ein vom Patienten ausgeschiedenes Gesamtvolumen an Urin ermittelt wird, mit
einer mittels eines Befestigungsmittels an einem Lagerpunkt, insbesondere am Krankenbett des Patienten, fixierbaren Halterung (12),
einem mit dem Katheter verbundenen Abflußschlauch (22),
einem mit dem Abschlußschlauch verbundenen Sammelgefäß (14), das freihängend an einem Haltemittel (36) aufhängbar ist,
einem elektrischen Kraftaufnehmer (38), der das Haltemittel (36) mit der Halterung verbindet,
Rechenmitteln, die den von dem Kraftaufnehmer ermittelten Wert um einen gespeicherten Wert für die Tara (14, 32, 34, 36) verringern und den so erhaltenen Wert durch einen ermittelten Wert dividieren, der der Wichte des Urins (40) entspricht, und
Anzeigemitteln zum Anzeigen der ermittelten Werte für gesammeltes Uringesamtvolumen (40) und durchschnittlichen Urinvolumenfluß in einem bestimmten Zeitintervall,
dadurch gekennzeichnet,
daß zwei in zwei senkrecht aufeinanderstehenden Ebenen angeordnete Winkelsensoren (42) vorgesehen sind, aus denen ein Signal für die Schieflage der Halterung (12) generiert wird, und daß in dem Rechenmittel (104) mittels trigonometrischer Funktionen jeweils für jede Ebene eine Korrektur des von dem Kraftaufnehmer (38) ermittelten Wertes vorgenommen wird, um die tatsächliche Gewichtskraft des an dem Kraftaufnehmer (38) freihängenden Sammelgefäßes (14, 32, 34, 36) und des in ihm gesammelten Urins (40) zu ermitteln, die der weiteren Berechnung zugrunde gelegt wird, und
daß eine Leitfähigkeitsmeßvorrichtung (74a-d) zur Ermittlung der Leitfähigkeit des Urins vorgesehen ist, wobei aus der Leitfähigkeit in den Rechenmitteln (104) mittels eines in einem Speicher abgelegten Zusammenhangs zwischen Leitfähigkeit und Dichte die Dichte des Urins ermittelt wird.

2. Urinmeßgerät nach Anspruch 1, dadurch gekennzeichnet, daß in den Rechenmitteln (104) der Wert für die Gewichtskraft des gesammelten Urins in einem ersten Schritt durch einen in einem weiteren Speicher abgelegten Wert für die durchschnittliche Erdbeschleunigung dividiert wird, und daß der so erhaltene Wert für die Masse des gesammelten Urins durch den Wert für die in den Rechenmitteln (104) ermittelte Dichte dividiert wird.

3. Urinmeßgerät nach Anspruch 1, dadurch gekennzeichnet, daß in den Rechenmitteln (104) die Dichte des Urins kontinuierlich ermittelt wird und aus der Gewichtszunahme des gesammelten Urins in einem bestimmten Zeitraum durch Dividieren durch die ermittelte Dichte des Urins auf die Volumenzunahme geschlossen wird.

4. Urinmeßgerät nach Anspruch 3, dadurch gekennzeichnet, daß das Urinmeßgerät weiter einen Zeitgeber (106) aufweist und daß in den Rechenmitteln (104) ein durchschnittlicher Volumenfluß an Urin in einem bestimmten Zeitintervall ermittelt wird, indem die ermittelte Volumenzunahme des Urins in dem Zeitintervall durch die Länge des Zeitintervalls dividiert wird.

5. Urinmeßgerät nach Anspruch 4, dadurch gekennzeichnet, daß das Urinmeßgerät einen Taktgeber (106) aufweist, der Zeitintervalle abmißt und daß die Ermittlung der Volumenzunahme und des durchschnittlichen Urinvolumenflusses in einem Zeitintervall jeweils automatisch durchgeführt wird.

6. Urinmeßgerät nach Anspruch 5, dadurch gekennzeichnet, daß in den Speichermitteln (104) Speicherplätze vorgesehen sind, in denen die durchschnittlichen Urinvolumenflüsse der jeweiligen Zeitintervalle abgespeichert werden können, und daß Mittel vorgesehen sind, um die einzelnen Werte nacheinander in einer alphanumerischen Anzeige (44) anzuzeigen.

7. Urinmeßgerät nach einem der vorhergehenden Ansprüche, dadurch gekennzeichnet, daß das Sammelgefäß (14) aus einem Kunststoffbeutel besteht.

8. Urinmeßgerät nach Anspruch 7, dadurch gekennzeichnet, daß das Sammelgefäß (14) an seinem oberen Rand mit einer Querleiste (32) versehen ist.

9. Urinmeßgerät nach Anspruch 8, dadurch gekennzeichnet, daß die Querleiste (32) aus Kunststoff besteht und einstückig mit einem bügelförmigen Haltemittel (36) verbunden ist.

10. Urinmeßgerät nach Anspruch 9, dadurch gekennzeichnet, daß Haltemittel (36) und Querleiste (32) einteilig als Kunststoffspritzgießteil ausgeführt sind.

11. Urinmeßgerät nach einem der vorhergehenden Ansprüche, dadurch gekennzeichnet, daß die Haltemittel (36) mit dem Kraftentnehmer (38) formschlüssig verriegelbar sind.

12. Urinmeßgerät nach einem der vorhergehenden Ansprüche, dadurch gekennzeichnet, daß das Sammelgefäß (14) als Einwegteil ausgebildet ist.

13. Urinmeßgerät nach einem der vorhergehenden Ansprüche, dadurch gekennzeichnet, daß das Sammelgefäß (14) mit einem Kunststoffschlauch (22) einteilig verschweißt ist.

14. Urinmeßgerät nach einem der vorhergehenden Ansprüche, dadurch gekennzeichnet, daß in den Abflußschlauch (22), der aus Kunststoff gefertigt ist, eine Tropfkammer (18) integriert ist.

15. Urinmeßgerät nach Anspruch 14, dadurch gekennzeichnet, daß die Tropfkammer (18) einen U-förmigen Kanal (60) aufweist, dessen einer senkrecht angeordneter Schenkel (60a) in der Tropfenfall-Linie der Tropfkammer (18) angeordnet ist und dessen anderer Schenkel zu einer Überlaufkammer (70) führt.

16. Urinmeßgerät nach Anspruch 14 oder 15, dadurch gekennzeichnet, daß die Tropfkammer (18) aus Kunststoff hergestellt ist und mit dem Abflußschlauch (22) verschweißt ist.

17. Urinmeßgerät nach einem der Ansprüche 14 bis 16, dadurch gekennzeichnet, daß Abflußschlauch (22), Tropfkammer (18) und Sammelgefäß (14) miteinander verschweißt sind und ein Einwegteil bilden.

18. Urinmeßgerät nach einem der Ansprüche 14 bis 17, dadurch gekennzeichnet, daß die Tropfkammer (18) ein Rückschlagventil (68) aufweist.

19. Urinmeßgerät nach einem der Ansprüche 14 bis 18, dadurch gekennzeichnet, daß die Tropfkammer (18) im wesentlichen quaderförmig ausgebildet ist.

20. Urinmeßgerät nach Anspruch 15, dadurch gekennzeichnet, daß der U-förmige Kanal (60) der Tropfkammer (18) durch in der Tropfkammer angeordnete Wände (62, 64) begrenzt wird.

21. Urinmeßgerät nach einem der Ansprüche 14 bis 20, dadurch gekennzeichnet, daß die vordere Wand (72) der Tropfkammer aus einem durchsichtigen Kunststoff besteht.

22. Urinmeßgerät nach einem der Ansprüche 14 bis 21, dadurch gekennzeichnet, daß das Urinmeßgerät ein Gehäuse (12) aufweist, in dem eine senkrecht angeordnete, kanalartige Einbuchtung (16) ausgebildet ist, in der die Tropfkammer (18) verriegelbar ist.

23. Urinmeßgerät nach einem der Ansprüche 14 bis 22, dadurch gekennzeichnet, daß auf der Rückseite der Tropfkammer (18) elastisch federnde Zungen (80) ausgebildet sind, die einen in der kanalartigen Einbuchtung (16) des Gehäuses angeordneten Befestigungssockel (82) einrastend umgreifen.

24. Urinmeßgerät nach Anspruch 22 oder 23, dadurch gekennzeichnet, daß sich die in dem Gehäuse (12) des Urinmeßgerätes (10) senkrecht angeordnete kanalartige Einbuchtung (16) rechts und links einer eingelegten Tropfkammer (18) zu Fingergriffmulden (24) erweitert.

25. Urinmeßgerät nach einem der Ansprüche 21 bis 24, dadurch gekennzeichnet, daß in der Wandung (64) des U-förmigen Kanals (60) der Tropfkammer (18) zumindest zwei Blechelektroden (74a-d) der Leitfahigkeitsmeßvorrichtung eingelassen sind.

26. Urinmeßgerät nach einem der Ansprüche 21 bis 24, dadurch gekennzeichnet, daß in zumindest einer Seitenwand (64) des U-förmigen Kanals der Tropfkammer vier Elektroden (74a-d) der Leitfahigkeitsmeßvorrichtung eingelassen sind.

27. Urinmeßgerät nach Anspruch 26, dadurch gekennzeichnet, daß die in eine Seitenwand (64) des U-förmigen Kanals eingelassenen Blechelektroden (74a-d) der Leitfahigkeitsmeßvorrichtung durch eine Hinterwand (59) der Tropfkammer (18) verlängert sind und auf der Rückseite der Tropfkammer federnde Kontaktzungen (76a-d) bilden.

28. Urinmeßgerät nach einem der Ansprüche 25 bis 27, dadurch gekennzeichnet, daß auf dem in der kanalartigen Einbuchtung (16) des Gehäuses (12) ausgebildeten Befestigungssockel (82) eine der Anzahl der Elektroden (74a-d) der Tropfkammer entsprechende Anzahl von Kontaktpunkten (90a-d) ausgebildet sind.

29. Urinmeßgerät nach Anspruch 28, dadurch gekennzeichnet, daß zumindest einer der elektrischen Kontaktpunkte (90a-d) des Befestigungssockels (82) des Gehäuses (12) gut wärmeleitend ausgebildet ist und mit einem Temperatursensor verbunden ist.

30. Urinmeßgerät nach Anspruch 29, dadurch gekennzeichnet, daß das von dem Temperatursensor gelieferte Signal in der Recheneinheit (104) mittels einer in einem Speicher abgelegten Tabelle zu einem Korrekturwert für die ermittelte Dichte des Urins verarbeitet wird.

31. Urinmeßgerät nach einem der Ansprüche 14 bis 30 dadurch gekennzeichnet, daß der die Tropfkammer (18) mit dem Sammelbeutel (14) verbindende Abschnitt des Abflußschlauchs (20) so lang bemessen ist, daß eine im wesentlichen kraftfreie Schlauchschlaufe entsteht.

32. Urinmeßgerät nach einem der Ansprüche 22 bis 31, dadurch gekennzeichnet, daß sich an den im Gehäuse (12) des Urinmeßgerätes im wesentlichen senkrecht angeordnete kanalartige Einbuchtung (16) für die Tropfkammer (18) ein den Abflußschlauch (22) aufnehmender Kanal (26) anschließt.

33. Urinmeßgerät nach Anspruch 32, dadurch gekennzeichnet, daß der den Abschlußschlauch (22) aufnehmende Kanal (26) im wesentlichen waagerecht angeordnet ist.

34. Urinmeßgerät nach einem der vorhergehenden Ansprüche, dadurch gekennzeichnet, daß das Urinmeßgerät als Stromversorgung eine Akkumulatorzelle aufweist.

35. Urinmeßgerät nach Anspruch 34, dadurch gekennzeichnet, daß das Gerät einen Taktgeber (106) aufweist, der die Stromversorgung der Recheneinheit (104) und/oder der Anzeigeeinheit (44) jeweils nach Ablauf eines durch den Taktgeber ermittelten Zeitintervalls einschaltet und nach Ermittlung der für das Zeitintervall relevanten Daten abschaltet.

36. Urinmeßgerät nach einem der Ansprüche 13 bis 35, dadurch gekennzeichnet, daß mit dem Abflußschlauch (22) ein Katheter verbunden ist, der ein Temperaturmeßelement aufweist, daß eine elektrische, mit dem Sensor verbundene Signalleitung mit dem Abflußschlauch (22) mechanisch verbunden oder in diesen integriert ist und daß in das Urinmeßgerät Mittel integriert sind, um das von dem Sensor gelieferte Meßsignal in eine Temperaturanzeige umzusetzen.

37. Verfahren zum automatischen Überwachen und Erfassen des Volumenflusses von Urin, die in einem an einer Halterung (12) freihängenden Sammelgefäß (14) des Gerätes aufgefangen werden und deren Gewicht ermittelt wird, wobei aus der Verknüpfung des Wertes für die Dichte des Urins und des Gewichtes auf den Volumenfluß des Urins geschlossen wird,
dadurch gekennzeichnet,
daß das ermittelte Gewicht einer Korrektur der Schieflage der Halterung mittels trigonometrischer Funktionen für jeweils zwei aufeinander senkrecht stehenden Ebenen unterworfen wird, in denen jeweils ein Winkelsensor (42) angeordnet ist, der ein Signal für die Schieflage generiert, wobei die elektrische Leitfähigkeit des Urins gemessen und daraus mittels eines empirisch ermittelten Zusammenhangs zwischen Leitfähigkeit und Dichte auf die Dichte des Urins geschlossen wird.

## Claims

1. A urine measuring device for the automatic monitoring and acquisition of the urine flow of a catheterised patient, with which, in particular, the total volume of urine secreted by the patient is determined, with
A holder (12), capable of being secured by means of securing media to a resting point, in particular to the patient's bed,
A drain hose (22) connected to the catheter,
A collector (14) connected to the drain hose, which can be freely suspended on a holding device (36),
An electrical transducer (38), which connects the holder (36) to the mount,
Computing media, which reduce the value determined by the transducer by a stored value for the tare weight (14, 32, 34, 36), and divides the value determined in this way by a determined value which corresponds to the weight of the urine (40), and
Display media for displaying the values determined for the collected urine total volume (40) and the mean volume flow in a specified interval of time,
characterised in that
Two angle sensors (42) arranged in two planes located perpendicular to one another are provided, from which a signal is generated for the offset position of the mount (12), and that in the calculator device (104), by means of trigonometric functions, in each case for each plane a correction is carried out for the values determined by the transducer (38), in order to determine the actual force of gravity of the collector (14, 32, 34, 36) suspended at the transducer (38) and to determine the urine (40) collected in it, which is used as the basis for the further calculation, and
That a conductivity measuring device (74a-d) is provided for the determination of the conductivity of the urine, in which situation the density of the urine is determined from the conductivity in the calculation media (104) by means of a connection, stored in a memory, between the conductivity and the density.

2. A urine measuring device according to Claim 1, characterised in that, in the calculating media (104), the value for the force of gravity of the collected urine is divided in a first step by a value deposited in another memory for the average gravitational acceleration, and that the value thus determined for the mass of the collected urine is divided by the value for the density determined in the calculator media (104).

3. A urine measuring device according to Claim 1, characterised in that, in the calculator media (104), the density of the urine is constantly determined, and, from the increase in weight of the collected urine in a specific period of time, a conclusion is reached regarding the increase in volume by dividing by the determined density value of the urine.

4. A urine measuring device according to Claim 3, characterised in that the urine measuring device also features a time sensor (106), and that, in the calculator media (104), a mean volume flow of urine in a specific interval of time is determined, inasmuch as the volume increase of the urine determined in the time interval is divided by the length of the time interval.

5. A urine measuring device according to Claim 4, characterised in that the urine measuring device features a cycle sensor (106) which measures time intervals, and that the determination of the volume increase and the mean urine volume flow in a time interval is carried out in each case automatically.

6. A urine measuring device according to Claim 5, characterised in that memory spaces are provided for in the memory media (104) in which the mean urine volume flows of the individual time intervals can be stored, and that media are provided in order to display the individual values one after another in an alphanumeric display (44).

7. A urine measuring device according to one of the foregoing Claims, characterised in that the collector (14) consists of a plastic bag.

8. A urine measuring device according to Claim 7, characterised in that the collector (14) is provided on its upper edge with a transverse strip (32).

9. A urine measuring device according to Claim 8, characterised in that the transverse strip (32) is made of plastic and is connected in one piece with a shackle-shaped holder (36).

10. A urine measuring device according to Claim 9, characterised in that holder (36) and transverse strip (32) are designed as a single piece, as a plastic injection-moulded component.

11. A urine measuring device according to one of the foregoing Claims, characterised in that the holder (36) is capable of being interlocked with positive fit with the transducer (38).

12. A urine measuring device according to one of the foregoing Claims, characterised in that the collector (14) is designed to be a disposable component.

13. A urine measuring device according to one of the foregoing Claims, characterised in that the collector (14) is welded as one piece to a plastic hose (22).

14. A urine measuring device according to one of the foregoing Claims, characterised in that a drip chamber (18) is integrated into the drain hose (22), which is made of plastic.

15. A urine measuring device according to Claim 14, characterised in that the drip chamber (18) features a U-shaped channel (60), of which one of the vertically arranged limbs (60a) is arranged i the drip fall line of the drip chamber (18), and of which the other limb leads to an overflow chamber.

16. A urine measuring device according to one of Claims 14 or 15, characterised in that the drip chamber (18) is made of plastic and is welded to the drain hose (22).

17. A urine measuring device according to one of Claims 14 to 16, characterised in that the drain hose (22), drip chamber (18), and collector (14) are welded to one another and form one disposable component.

18. A urine measuring device according to one of Claims 14 to 17, characterised in that the drip chamber (18) features a non-return valve (68).

19. A urine measuring device according to one of Claims 14 to 18, characterised in that the drip chamber (18) is designed to be essentially quadratic in shape.

20. A urine measuring device according to Claim 15, characterised in that the U-shaped channel (60) ofthe drip chamber (18) is delimited by the walls (62, 64) arranged in the drip chamber.

21. A urine measuring device according to one of Claims 14 to 20, characterised in that the front wall (72) of the drip chamber consists of transparent plastic.

22. A urine measuring device according to one of Claims 14 to 21, characterised in that the urine measuring device features a mount (12), in which an indentation (16) is formed, arranged vertically and in channel shape, in which the drip chamber (18) is capable of being locked in place.

23. A urine measuring device according to one of Claims 14 to 22, characterised in that, on the rear of the drip chamber (18) elastic spring-loaded tongues (80) are formed, which encompass and engage with a securing base (82) arranged in the channel-shaped indentation (16) of the housing.

24. A urine measuring device according to one of Claims 14 to 23, characterised in that the indentation (16) in channel shape, arranged vertically in the housing (12) of the urine measuring device (10), extends to the left and right of an inserted drip chamber (18) to form finger grip depressions.

25. A urine measuring device according to one of Claims 14 to 23, characterised in that at least two plate electrodes (74a-d) of the conductivity measuring device are let into the wall (64) of the U-shaped channel (60) of the drip chamber (18).

26. A urine measuring device according to one of Claims 14 to 24, characterised in that four electrodes (74a-d) of the conductivity measuring device are let into at least one side wall (64) of the U-shaped channel of the drip chamber.

27. A urine measuring device according to Claim 26, characterised in that plate electrodes (74a-d) of the conductivity measuring device, let into one side wall (64) of the U-shaped channel, are extended through a rear wall (59) of the drip chamber (18), and form spring-loaded contact tongues (76a-d) on the rear face of the drip chamber.

28. A urine measuring device according to one of Claims 25 to 27, characterised in that, on the securing base (82) formed on the channel-shaped indentation (16) of the housing (12), a number of contact points are formed (90a-d) which correspond to the number of electrodes (74a-d) of the drip chamber.

29. A urine measuring device according to Claim 28, characterised in that at least one of the electrical contact points (90a-d) of the securing base (82) of the housing (12) is designed to be a good thermal conductor, and is provided with a temperature sensor.

30. A urine measuring device according to Claim 29, characterised in that the signal supplied by the temperature sensor is processed in the calculator unit (104) by means of a table deposited in a memory to provide a correction value for the determined density of the urine.

31. A urine measuring device according to one of Claims 14 to 30, characterised in that the section of the drain hose (20) connecting the drip chamber (18) to the collector (14) is designed to be sufficiently long for an essentially force-free hose loop to be created.

32. A urine measuring device according to one of Claims 22 to 31, characterised in that a channel (26) accommodating the drain hose (22) is connected to the channel-shaped indentation (16) for the drip chamber (18), essentially arranged vertically in the housing (12) of the urine measuring device.

33. A urine measuring device according to Claim 32, characterised in that the channel (26) accommodating the drain hose (22) is arranged essentially horizontal.

34. A urine measuring device according to one of the foregoing Claims, characterised in that the urine measuring device features a battery cell as its power supply.

35. A urine measuring device according to Claim 34, characterised in that the device features a time sensor (106) which switches on he power supply to the calculator unit (104) and/or the display unit (44) in each case after the expiry of the interval of time determined by the time sensor, and switches it off again after the determination of the data which is relevant for the time interval.

36. A urine measuring device according to one of Claims 13 to 3 5, characterised in that a catheter is connected to the drain hose (22), the said catheter featuring a temperature measuring element; that an electrical signal lead connected to the sensor is mechanically connected to the drain hose (22), or is integrated in it; and that media are integrated in the urine measuring device in order to convert the measurement signal supplied by the sensor into a temperature display.

37. A process for the automatic monitoring and acquisition of the volume flow of urine, which is caught in a collector (14) of the device, hanging freely on a mount (12), and the weight of which is determined, in which context a conclusion is drawn on the volume flow of the urine from the linking of the value for the density of the urine and the weight,
characterised in that
The weight to be determined is subjected to a correction of the tilt of the mount by means of trigonometric function for two planes in each case, standing perpendicular to one another, in which, in each case, an angle sensor (42) is arranged, which generates a signal for the tilt position, in which context the electrical conductivity of the urine is measured, and, from this, a conclusion is reached about the density of the urine by means of an empirically-determined connection between conductivity and density.

## Revendications

1. Appareil de mesure de l'urine pour la surveillance et l'enregistrement automatiques du débit urinaire d'un patient auquel on a appliqué un cathéter, dans lequel on détermine en particulier un volume total d'urine excrété par le patient, comprenant
un dispositif de support (12) qui peut être fixé à l'aide d'un moyen de fixation à un point d'appui, en particulier au lit d'hôpital sur lequel est couché le patient,
un tuyau d'écoulement (22) relié au cathéter,
un récipient collecteur (14) relié au tuyau d'écoulement, qui peut être suspendu librement à un moyen de retenue (36),
un transducteur de force électrique (38) qui relie le moyen de retenue (36) au dispositif de support,
des calculateurs qui soustraient de la valeur déterminée par le transducteur de force une valeur mémorisée pour la tare (14, 32, 34, 36) et qui divisent la valeur ainsi obtenue par une valeur déterminée qui correspond au poids spécifique de l'urine (40), et
un dispositif d'affichage pour afficher les valeurs déterminées pour le volume total récolté de l'urine (40) et pour le débit volumique urinaire moyen dans un intervalle de temps déterminé,
caractérisé
en ce qu'on prévoit deux détecteurs (42) de la position angulaire disposés dans deux plans mutuellement perpendiculaires, à partir desquels est généré un signal pour la position d'inclinaison du dispositif de support (12), et en ce qu'on procède dans le calculateur (104), au moyen de fonctions trigonométriques, respectivement pour chaque plan, à une correction de la valeur déterminée par le transducteur de force (38) pour déterminer le poids réel du récipient collecteur (14, 32, 34, 36) suspendu librement au transducteur de force (38) et de l'urine (40) récoltée dans ce dernier, qui sert de base au calcul ultérieur, et
en ce qu'on prévoit un dispositif de mesure de la conductibilité (74a-d) pour déterminer la conductibilité de l'urine, dans lequel, à partir de la conductibilité, on détermine la masse volumique de l'urine dans les calculateurs (104) au moyen d'un rapport entre la conductibilité et la masse volumique placé dans une mémoire.

2. Appareil de mesure de l'urine selon la revendication 1, caractérisé en ce qu'on divise, dans les calculateurs (104), la valeur pour le poids de l'urine récoltée dans une première étape par une valeur mise dans une mémoire supplémentaire pour l'accélération moyenne due à la gravité et en ce qu'on divise la valeur ainsi obtenue pour la masse de l'urine récoltée par la valeur pour la masse volumique déterminée dans les calculateurs (104).

3. Appareil de mesure de l'urine selon la revendication 1, caractérisé en ce qu'on détermine la masse volumique de l'urine en continu dans les calculateurs (104) et on tire des conclusions quant à l'augmentation du volume à partir de l'augmentation du poids de l'urine récoltée dans un laps de temps déterminé en divisant par la masse volumique déterminée de l'urine.

4. Appareil de mesure de l'urine selon la revendication 3, caractérisé en ce que l'appareil de mesure de l'urine présente en outre un générateur de rythme (106) et en ce qu'on détermine, dans les calculateurs (104), un débit volumique moyen de l'urine dans un intervalle de temps déterminé dans lequel on divise l'augmentation en volume déterminée de l'urine dans l'intervalle de temps par la longueur de l'intervalle de temps.

5. Appareil de mesure de l'urine selon la revendication 4, caractérisé en ce que l'appareil de mesure de l'urine présente un générateur de rythme (106) qui mesure l'intervalle de temps et en ce qu'on effectue la détermination de l'augmentation du volume et du débit volumique urinaire moyen dans un intervalle de temps respectivement de manière automatique.

6. Appareil de mesure de l'urine selon la revendication 5, caractérisé en ce qu'on prévoit, dans les ordinateurs (104), des endroits de mémoire dans lesquels on peut mémoriser les débits volumiques urinaires moyens des intervalles de temps respectifs et en ce qu'on prévoit des moyens pour afficher les valeurs individuelles successivement sur un écran alphanumérique (44).

7. Appareil de mesure de l'urine selon l'une quelconque des revendications précédentes, caractérisé en ce que le récipient collecteur (14) est constitué d'un sac en matière synthétique.

8. Appareil de mesure de l'urine selon la revendication 7, caractérisé en ce que le récipient collecteur (14) est muni, sur son bord supérieur, d'une nervure transversale (32).

9. Appareil de mesure de l'urine selon la revendication 8, caractérisé en ce que la nervure transversale (32) est constituée d'une matière synthétique et est reliée en une seule pièce avec un moyen de retenue (36) en forme d'étrier.

10. Appareil de mesure de l'urine selon la revendication 9, caractérisé en ce que le moyen de retenue (36) et la nervure transversale (32) sont réalisés en une seule pièce sous forme d'un élément en matière synthétique moulé par injection.

11. Appareil de mesure de l'urine selon l'une quelconque des revendications précédentes, caractérisé en ce que le moyen de retenue (36) peut être verrouillé mécaniquement avec le transducteur de force (38).

12. Appareil de mesure de l'urine selon l'une quelconque des revendications précédentes, caractérisé en ce que le récipient collecteur (14) est réalisé sous orme d'un élément à jeter.

13. Appareil de mesure de l'urine selon l'une quelconque des revendications précédentes, caractérisé en ce que le récipient collecteur (14) est soudé en une seule pièce avec un tuyau flexible (22) en matière synthétique.

14. Appareil de mesure de l'urine selon l'une quelconque des revendications précédentes, caractérisé en ce qu'on intègre, dans le tuyau d'écoulement (22) qui est réalisé en une matière synthétique, une chambre d'égouttage (18).

15. Appareil de mesure de l'urine selon la revendication 14, caractérisé en ce que la chambre d'égouttage (18) présente un canal en U (60) dont une branche (60a) disposée verticalement vient s'appliquer dans la ligne de la chambre d'égouttage (18) suivie par la chute des gouttes et dont l'autre branche mène à une chambre de trop-plein (70).

16. Appareil de mesure de l'urine selon la revendication 14 ou 15, caractérisé en ce que la chambre d'égouttage (18) est fabriquée en une matière synthétique et est soudée au tuyau d'écoulement (22).

17. Appareil de mesure de l'urine selon l'une quelconque des revendications 14 à 16, caractérisé en ce que le tuyau d'écoulement (22), la chambre d'égouttage (18) et le récipient collecteur (14) sont soudés l'un à l'autre et forment un élément à jeter.

18. Appareil de mesure de l'urine selon l'une quelconque des revendications 14 à 17, caractérisé en ce que la chambre d'égouttage (18) présente une soupape anti-retour (68).

19. Appareil de mesure de l'urine selon l'une quelconque des revendications 14 à 18, caractérisé en ce que la chambre d'égouttage (18) est réalisée sous une forme essentiellement parallélipipédique.

20. Appareil de mesure de l'urine selon la revendication 15, caractérisé en ce que le canal en U (60) de la chambre d'égouttage (18) est délimité par des parois (62, 64) disposées dans la chambre d'égouttage.

21. Appareil de mesure de l'urine selon l'une quelconque des revendications 14 à 20, caractérisé en ce que la paroi antérieure (72) de la chambre d'égouttage est constituée d'une matière synthétique translucide.

22. Appareil de mesure de l'urine selon l'une quelconque des revendications 14 à 21, caractérisé en ce que l'appareil de mesure de l'urine présente un logement (12) dans lequel est réalisé un renfoncement (16) en forme de canal disposé verticalement, dans lequel on peut verrouiller la chambre d'égouttage (18).

23. Appareil de mesure de l'urine selon l'une quelconque des revendications 14 à 22, caractérisé en ce que des languettes résilientes (80) sont réalisées sur le coté dorsal de la chambre d'égouttage (18), qui enserrent par encliquetage un socle de fixation (82) disposé dans le renfoncement (16) du logement en forme de canal.

24. Appareil de mesure de l'urine selon la revendication 22 ou 23, caractérisé en ce que le renfoncement (16) en forme de canal disposé verticalement dans le logement (12) de l'appareil de mesure (10) de l'urine s'élargit à droite et à gauche d'une chambre d'égouttage encastrée (18) pour former des cavités de préhension (24) pour les doigts.

25. Appareil de mesure de l'urine selon l'une quelconque des revendications 21 à 24, caractérisé en ce qu'au moins deux électrodes à plaques (74a-d) du dispositif de mesure de la conductibilité sont encastrées dans la paroi (64) du canal en U (60) de la chambre d'égouttage (18).

26. Appareil de mesure de l'urine selon l'une quelconque des revendications 21 à 24, caractérisé en ce que quatre électrodes (74a-d) du dispositif de mesure de la conductibilité sont encastrées dans au moins une paroi latérale (64) du canal en U de la chambre d'égouttage.

27. Appareil de mesure de l'urine salon la revendication 26, caractérisé en ce que les électrodes à plaques (74a-d) du dispositif de mesure de la conductibilité encastrées dans une paroi latérale (64) du canal en U se prolongent à travers une paroi arrière (59) de la chambre d'égouttage (18) et forment des languettes de contact résilientes (76a-d) sur le côté dorsal de la chambre d'égouttage.

28. Appareil de mesure de l'urine selon l'une quelconque des revendications 25 à 27, caractérisé en ce que, sur le socle de fixation (82) réalisé dans le renfoncement (16) du logement (12) en forme de canal, sont réalisés un certain nombre de points de contact (90a-d) correspondant au nombre des électrodes (74a-d) de la chambre d'égouttage.

29. Appareil de mesure de l'urine selon la revendication 28, caractérisé en ce qu'au moins un des points de contact électriques (90a-d) du socle de fixation (82) du logement (12) est réalisé pour être un bon conducteur à la chaleur et est relié à un capteur de température.

30. Appareil de mesure de l'urine selon la revendication 29, caractérisé en ce que le signal émis par le capteur de température est traité dans l'unité de calcul (104) au moyen d'un tableau mis en mémoire par rapport à une valeur de correction pour la masse volumique déterminée de l'urine.

31. Appareil de mesure de l'urine selon l'une quelconque des revendications 14 à 30, caractérisé en ce que la dimension en longueur de la section du tuyau d'écoulement (20) reliant la chambre d'égouttage (18) au sac collecteur (14) est telle que l'on obtient une boucle de tuyau essentiellement exempta de force.

32. Appareil de mesure de l'urine selon l'une quelconque des revendications 22 à 31, caractérisé en ce qu'au renfoncement (16) destiné à la chambre d'égouttage (18), en forme de canal et disposé essentiellement à la verticale dans le logement (12) de l'appareil de mesure de l'urine, se raccorde un canal (26) dans lequel vient se loger le tuyau d'écoulement (22).

33. Appareil de mesure de l'urine selon la revendication 32, caractérisé en ce que le canal (26) dans lequel vient se loger le tuyau d'écoulement (22) est disposé essentiellement à l'horizontale.

34. Appareil de mesure de l'urine selon l'une quelconque des revendications précédentes, caractérisé en ce que l'appareil de mesure de l'urine présente un élément d'accumulateur à titre d'alimentation électrique.

35. Appareil de mesure de l'urine selon la revendication 34, caractérisé en ce que l'appareil présente un générateur de rythme (106) qui met en circuit l'alimentation électrique de l'unité de calcul (104) et/ou de l'unité d'affichage (44), respectivement après l'écoulement d'un intervalle de temps déterminé par le générateur de rythme, et met l'alimentation électrique hors-circuit après détermination des données pertinentes pour l'intervalle de temps.

36. Appareil de mesure de l'urine selon l'une quelconque des revendications 13 à 35, caractérisé en ce qu'un cathéter est relié au tuyau d'écoulement (22), qui présente un élément de mesure de la température, en ce qu'un circuit électrique d'acheminement des signaux relié au capteur est relié mécaniquement au tuyau d'écoulement (22) ou est intégré dans ce dernier, et en ce que des moyens sont intégrés dans l'appareil de mesure de l'urine pour convertir le signal de mesure émis par le capteur en une indication de température.

37. Procédé pour la surveillance et l'enregistrement automatiques du débit volumique de l'urine qui est récoltée dans un récipient collecteur (14) de l'appareil suspendu librement à un dispositif de support (12) et dont on détermine le poids, par lequel on tire des conclusions quant au débit volumique de l'urine à partir de la relation entre la valeur pour la masse volumique de l'urine et celle pour le poids,
caractérisé en ce que
on soumet le poids déterminé à une correction de la position d'inclinaison du dispositif de support en utilisant des fonctions trigonométriques pour respectivement deux plans mutuellement perpendiculaires dans lesquels est disposé respectivement un détecteur (42) de la position angulaire qui génère un signal pour la position d'inclinaison, dans lequel on mesure la conductibilité électrique de l'urine et on en tire des conclusions quant à la masse volumique de l'urine à l'aide d'un rapport déterminé par voie empirique entre la conductibilité et la masse volumique.
